# EUROPÄISCHE PATENTSCHRIFT

(11) **EP 3 709 968 B1**
(45) Veröffentlichungstag und Bekanntmachung des Hinweises auf die Patenterteilung: **23.08.2023**
(21) Anmeldenummer: 18799563.4
(22) Anmeldetag: 14.11.2018
(51) Int. Cl.: A61K 9/00, A61K 45/06, A61K 47/10, A61K 47/18, A61K 9/06, A61K 9/107, A61K 31/14, A61K 31/196, A61K 31/4174, A61K 31/496, A61K 31/665, A61K 31/7056, A61K 31/192, A61Q 7/00, A61P 31/04, A61P 31/10, A61P 29/00, A61P 15/02, A61K 31/4706

(54) **EMULSIONEN ZUR BEHANDLUNG VON SCHLEIMHAUTINFEKTIONEN**
EMULSIONS FOR THE TREATMENT OF VAGINAL INFECTIONS
ÉMULSIONS DESTINÉS AU TRAITEMENT D'INFECTIONS VAGINALES

(30) Priorität: 14.11.2017 EP 17201650; 14.11.2017 EP 17201651
(43) Veröffentlichungstag der Anmeldung: 23.09.2020
(73) Patentinhaber: ProFem GmbH, 1180 Wien (AT)
(72) Erfinder: NOE, Marion, 1180 Wien (AT); NOE, Christian, 1180 Wien (AT)
(74) Vertreter: SONN Patentanwälte OG
(86) Internationale Anmeldenummer: PCT/EP2018/081253
(87) Internationale Veröffentlichungsnummer: WO 2019/096857

(56) Entgegenhaltungen:
- EP-A2- 0 923 937
- WO-A2-02/078648
- WO-A2-2007/131253
- US-A- 5 686 089
- US-A1- 2010 256 238
- MENDLING WERNER ET AL: "Use of locally delivered dequalinium chloride in the treatment of vaginal infections: a review", ARCHIVES OF GYNECOLOGY AND OBSTETRICS, SPRINGER VERLAG, BERLIN, DE, Bd. 293, Nr. 3, 27. Oktober 2015 (2015-10-27), Seiten 469-484, XP035879005, ISSN: 0932-0067, DOI: 10.1007/S00404-015-3914-8 [gefunden am 2015-10-27]

## Beschreibung

Die Erfindung betrifft Emulsionen zur Behandlung von Schleimhautinfektionen, insbesondere von entzündlichen Scheideninfektionen.

Die WO 2007/131253 A2 betrifft die Verwendung von einem antimykotischen Wirkstoff und einem Epithelzellen- oder Endothelzellen-Adhäsionsinhibitor zur Herstellung eines Kombinationsarzneimittels zur topischen Behandlung von Candidamykosen ausgewählt aus vulvovaginaler Candidiasis, oropharyngealer Candidiasis (Mundsoor), Windeldermatitis (Windelsoor) und intertriginösem Ekzem.

Die WO 02/0768648 A2 betrifft pharmazeutische Zusammensetzungen zur topischen Anwendung, enthaltend ein Antimykotikum, z.B. Terbinafin, und einen zweiten Wirkstoff, z.B. Diclofenac oder Indomethacin. Die Zusammensetzungen können zur Vorbeugung oder Behandlung von Pilzinfektionen, vor allem durch Dermatophyten, eingesetzt werden.

Die US 5 686 089 A betrifft pharmazeutische Zusammensetzungen zur topischen Anwendung, die einen antimikrobiellen Wirkstoff und eine feuchtigkeitsspendende Komponente enthalten. Die Zusammensetzungen können z.B. bei vaginalen Pilzinfektionen zum Einsatz kommen.

Mendling et al. (Mendling, Werner, et al. "Use of locally delivered dequalinium chloride in the treatment of vaginal infections: a review." Archives of gynecology and obstetrics 293.3 (2016): 469-484) beschreibt Behandlungsmöglichkeiten von vaginalen Infektionen mit Dequaliniumchlorid.

Die vorliegende Erfindung stellt sich zur Aufgabe, verbesserte NSAID Präparate zur Verfügung zu stellen, die besonders gut zur Behandlung von Schleimhautinfektionen und -entzündungen geeignet sind. Insbesondere, ist es eine Aufgabe der Erfindung, Kombinationspräparate aus einem Antimykotikum und einem NSAID zur Verfügung zu stellen, die besonders gut geeignet zur topischen Behandlung von vaginalen und dermalen Pilzinfektionen sind. Derartige Emulsionen sollen auch bei hartnäckigen Pilzinfektionen, welche besonders im Vaginalbereich auftreten, wirksam sein, sowie mikrobiologisch und chemisch stabil sein.

Demgemäß betrifft die vorliegende Erfindung eine Emulsion, vorzugsweise in Form einer Salbe oder einer Creme, welche eine wässerige Phase und eine Ölphase (Fettphase) umfasst, enthaltend ein Antimykotikum und ein NSAID, dadurch gekennzeichnet, dass (a) das NSAID Diclofenac in einer Konzentration von 0,1 bis 0,5 Gewichtsprozent, Indometacin in einer Konzentration von 0,1 bis 0,4 Gewichtsprozent, Naproxen in einer Konzentration von 1 bis 5 Gewichtsprozent, Ibuprofen in einer Konzentration von 0,5 bis 2,5 Gewichtsprozent, Dexibuprofen in einer Konzentration von 0,25 bis 1,25 Gewichtsprozent, Ketoprofen in einer Konzentration von 0,25 bis 1,25 Gewichtsprozent, Mefenaminsäure in einer Konzentration von 0,5 bis 4 Gewichtsprozent, oder Lornixocam in einer Konzentration von 0,02 bis 0,04 Gewichtsprozent ist, wobei das NSAID in Salzform vorliegt; dass (b) das Gewichtsverhältnis der Wasser- zur Ölphase in dieser Emulsion zwischen den Werten 2,0 und 2,7 liegt, wobei das Gewichtsverhältnis unter Einbeziehen der in den Phasen gelösten Stoffen berechnet wird, wobei Emulgatoren weder der Wasser- noch der Ölphase zugerechnet werden, und dass (c) der pH Wert der Emulsion nicht unter dem Wert 6,5 und nicht über 8,5 vorzugsweise im Bereich 7,0 bis 8,0 liegt, vorzugsweise zur Behandlung von dermalen und vaginalen Pilzinfektionen. Die Erfindung betrifft auch die genannte Emulsion zur Anwendung in der Behandlung von vaginalen Pilzinfektionen, insbesondere chronischen vaginalen Pilzinfektionen, vorzugsweise wobei die Behandlung eine topische Behandlung ist.

Weiterhin betrifft die vorliegende Erfindung Verfahren zur Herstellung der genannten Emulsion gemäß den unabhängigen Ansprüchen 12-15.

Die vorliegende Erfindung stellt verbesserte Präparate der eingangs beschriebenen Art zur Verfügung. Insbesondere zeigen die erfindungsgemäßen Präparate neben der optimierten Pharmakokinetik durch den Angriff unmittelbar am Infektionsort auch eine optimale Pharmakodynamik. Die erfindungsgemäßen Präparate ermöglichen eine ausreichende Wirksamkeit der NSAIDs bei Einsatz niedriger - und damit nebenwirkungsarmer - Konzentrationen. Im Fall der erfindungsgemäßen Kombinationspräparate wird damit nicht nur eine besonders gute Wirksamkeit des Antimykotikums ermöglicht, sondern auch das Zusammenwirken mit dem NSAID signifikant verbessert, indem durch eine niedrige Konzentration an NSAID trotzdem eine ausreichende Wirksamkeit gesichert wird, ohne die dabei auftretenden Nebenwirkungen (Reizungen, Brennen, etc.) in Kauf nehmen zu müssen. In der Praxis führt dies dazu, dass zuvor aus diesen Nebenwirkungsgründen nicht einsetzbare Kombinationen aus Antimykotikum und NSAID mit der Lehre der vorliegenden Erfindung den Patientinnen zugänglich gemacht werden können und diese Patientinnen nunmehr einer erfolgreichen Therapie zugeführt werden können. Erfindungsgemäß hat sich aber gezeigt, dass für eine optimale pharmakodynamische Wirkung die Konzentration des NSAID in der wässerigen Phase von entscheidender Bedeutung ist. Die Verfügbarmachung des eingebrachten NSAIDs wird durch das Zusammenwirken des Herstellungsprozesses, des Wasser/Öl-Verhältnisses und des pH-Wertes, durch welchen das überwiegende Vorliegen des NSAIDs in seiner Salzform gewährleistet wird, erreicht.

Die NSAIDs, die erfindungsgemäß zum Einsatz kommen, sind ausgewählt aus der Gruppe, bestehend aus Diclofenac, Ibuprofen, Dexibuprofen, Ketoprofen, Lornoxicam, Mefenaminsäure, Naproxen und Indometacin. Die in für die topische Therapie zugelassenen Medikamenten übliche Konzentration, in der Diclofenac eingesetzt wird, liegt im Bereich von 1 bis 2% (10 mg/bzw. 20 mg/g). Erfindungsgemäß wird Diclofenac in einer Konzentration von 0,1% bis 0,5 % (1 - 5 mg pro g Creme) eingesetzt, vorzugsweise von 0,2% bis 0,35%. Die in zur topischen Therapie zugelassenen Medikamenten übliche Konzentration, in der Ibuprofen eingesetzt wird, liegt bei 5% (50 mg/g Creme/Gel). Erfindungsgemäß wird Ibuprofen eingesetzt in einer bevorzugten Menge von 0,5 bis 2,5% (5 - 25 mg pro g Creme), vorzugsweise von 1 bis 2%. Weitere Beispiele für bevorzugte NSAIDs und deren bevorzugte Mengen ("Erfindungsgemäße Konzentration") in Relation zu den 'üblichen' Dosen können aus Tabelle A ersehen werden.

**Tabelle A:**

| Wirkstoff | Einzeldosis | Tageshöchstdosis | Beispiel | Einzeldosis | Tageshöchstdosis | Beispiel | **Erfindungsgemäße Konzentration** |
|---|---|---|---|---|---|---|---|
| | oral/i.v. | | | topisch | | | |
| Diclofenac Natrium | 50 - 75 mg | 150 - 225 mg | Voltaren 50 mg Tabletten | | | Voltadol Schmerzgel 1%, 10 mg/g | **0,2 - 0,5 Gew%** |
| | | | 75 mg Amp. | 40 - 80 mg | 240 mg | 2%, 20 mg/g | |
| Indometacin | 25 - 75 mg | 50 - 150 mg | Indocid 25 mg | 20-40 mg | 40-80 mg | IndometGel | **0,1 - 0,4 Gew.%** |
| | | | 75 mg ret. | | | 1%, 10 mg/g | |
| Naproxen | 250 - 500 mg | 1000 mg | Naproxen FT 250/500 mg Naproxen Susp. 50 mg/ml | | | | **0,5 - 2,5 Gew%** |
| Ibuprofen | 200 - 800 mg | 2400 mg | Ibuprofen FT 200/ 400/800 mg | | | Ibutop Creme/ Gel | |
| | | | | 100 -250 mg | 1000 mg | 5%, 5g/100 g | **0,5-2,5 Gew.%** |

Im Rahmen der Erfindung sind die folgenden NSAIDs vorgesehen:

| **NSAID** | **Bevorzugte Konzentration (Gew. %)** | **Bevorzugte Einzeldosis (mg)** |
|---|---|---|
| Diclofenac | 0,1 - 0,5 (vorzugsweise 0,2 - 0,4) | 4 bis 8 mg |
| Indometacin | 0,1 - 0,4 | 2 - 8 mg |
| Naproxen | 1 - 5 | 20 bis 100 mg |
| Ibuprofen | 0,5 - 2,5 | 10 bis 50 mg |
| Dexibuprofen | 0,25-1,25 | 5 bis 25 mg |
| Ketoprofen | 0,25-1,25 | 5 bis 25 mg |
| Mefenaminsäure | 0,5 - 4 | 10 - 40 mg |
| Lornixocam | 0,02 - 0,04 | 0,4 bis 0,8 mg |

Es hat sich erfindungsgemäß gezeigt, dass die Behandlung von Pilzerkrankungen, insbesondere von Candidamykosen, besonders effektiv mit Hilfe eines Kombinationspräparates aus einem Antimykotikum mit einem Arzneistoff erfolgen kann, welcher zugleich auf die Adhäsion der Mikroorganismen Einfluss nimmt. Die Arzneimittel werden vorteilhaft topisch appliziert, weil dadurch der Wirkort unmittelbar erreicht wird und zugleich eine minimale systemische Belastung erfolgt. Solche Wirkstoffkombinationen sind in den Patenten, die aus der WO 2007/131253 A2 abgeleitet sind, geschützt.

Gegenstand der Erfindung sind speziell für die vaginale Applikation besonders geeignete Medikamentenkombinationen, sowie ihre Herstellung und Verwendung. Durch Beachtung der besonderen physiologischen und pathophysiologischen Gegebenheiten in der Scheide erweisen sich sowohl eine spezielle Zusammensetzung als auch ein spezieller Herstellungsprozess als besonders vorteilhaft. Die Beachtung spezifischer Parameter führt dazu, dass bei der Therapie von vaginalen Scheideninfektionen ein besonders guter therapeutischer Effekt erreicht wird.

Bezüglich der adhäsionshemmenden Komponente betrifft die vorliegende Verbindung nur die Gruppe der NSAIDs (non-steroidal antiinflammatoric drugs), weil Arzneistoffe dieser Gruppe neben der antiadhäsiven Wirkung, welche die Anhaftung verhindern, auch eine schmerzlindernde und entzündungshemmende Wirkung haben. Beide Effekte, vor allem der entzündungshemmende Effekt, sind vor allem bei rezidivierenden Formen von Candidamykosen sehr willkommen, weil diese mit chronischer Entzündung und starken Schmerzen verbunden sind.

Vorgesehene NSAIDs sind dabei Diclofenac, Ibuprofen, Dexibuprofen Ketoprofen, Mefenaminsäure, Naproxen, Lornoxicam und Indometacin. Bevorzugte Antimykotika sind Nystatin, Ciclopirox oder Ciclopiroxolamin, oder Antimykotika aus der Gruppe der Azole (Imidazole, Triazole, Tetraazole) wie Clotrimazol, Fluconazol, Miconazol, Itraconazol, Tioconazol, Voriconazol, Bifonazol, Econazol, Isoconazol, Fenticonazol, Sertaconazol, Ketoconazol, Posaconazol, Quilseconazol, Oteseconazol (VT-1161), von Ibrexafungerp (SCY-078)..

### Strukturformel von Ibrexafungerp (SCY-078)

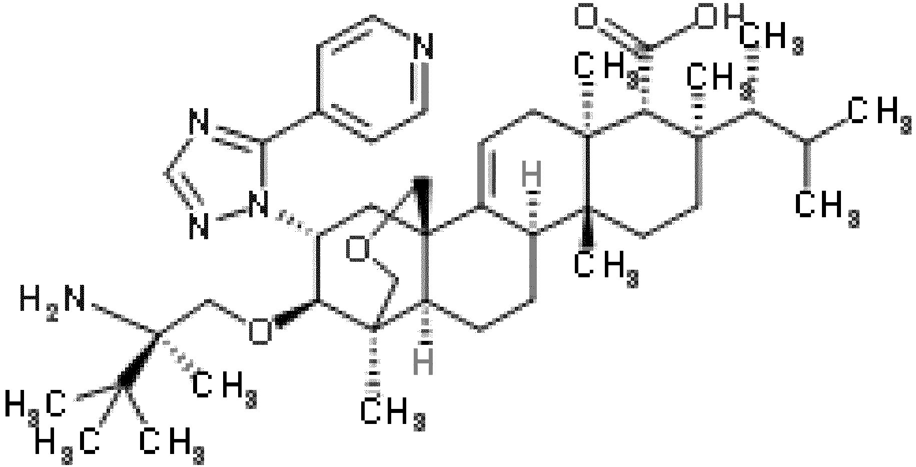

### Strukturformel von Oteseconazol (VT-1161)

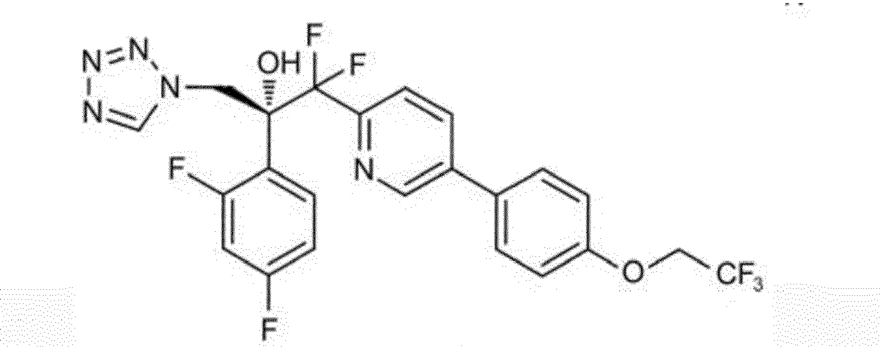

Überraschender Weise hat sich bei der Anwendung gezeigt, dass zur Erzielung einer optimalen Wirkung dem Verhältnis der Anteile des Antimykotikums und des NSAIDs eine besondere Bedeutung zukommt. In den Kombinationen eines Antimykotikums mit einem NSAID kann das Antimykotikum in üblichen

Dosierungen eingesetzt werden (Clotrimazol 1-10%, Ketokonazol 1-5%, bevorzugt 2%, Nystatin 100.000 IE/ml bzw g). Da das NSAID seine Wirkung in gleicher Weise am Erreger in der Scheide und am Scheidenepithel ausübt, ist zum einen die topische Applikation des Arzneimittels, bei welcher es ja direkt am Wirkort aufgebracht wird, deutlich einer systemischen Anwendung überlegen, zum anderen sollte deshalb das NSAID in weit niedrigeren Dosen zum Einsatz kommen, als bei den üblichen systemischen Anwendungen als Schmerzmittel. Bei der vaginalen Anwendung wird ein therapeutischer Effekt bereits ab einem Zehntel, vorzugsweise einem Fünftel einer Wirkstoffkonzentration erreicht, welche bei üblichen dermalen Formulierungen von NSAID zur Anwendung kommen (im Falle von Diclofenac 200 bis 500 mg/100g Salbe). Die niedrige Wirkstoffkonzentration ist auch deshalb besonders vorteilhaft, weil die systemische Aufnahme des NSAID vernachlässigbar wird und keinerlei Gefahr systemischer Nebenwirkungen besteht. Zugleich muss beachtet werden, dass es bei der topischen Applikation auf Schleimhäuten leicht zur Überdosierung des NSAID kommen kann (z.B. ist eine Creme mit Diclofenac: ab 0,5 % schmerzhaft und der rasche Schmerzlinderungseffekt tritt nicht mehr auf). Vor der Anwendung am Markt befindlicher NSAID-hältiger Cremes (1-2% Diclofenac, 5% Ibuprofen) auf Schleimhäuten wird in den jeweiligen Gebrauchsinformationen gewarnt (z.B. Gebrauchsinformationen Voltaren Emulgel, Ibutop-Gel), weil es zur Reizung der Schleimhäute kommt, welche dem antiinflammatorischen Effekt entgegenwirkt. Es sollte daher erfindungsgemäß das NSAID in keiner größeren Menge als 50% der üblichen niedrigsten Formulierung für dermale Anwendungen eingesetzt werden. Bei dieser Konzentration können bereits Reizungen auftreten. Damit bewegt sich die einzusetzende Wirkstoffkonzentration der NSAID vorzugsweise in einem relativ kleinen therapeutischen Fenster von 10 - 60, vorzugsweise von 20 - 40% des für topische Formulierungen üblichen Wertes. Demgemäß enthält eine besonders bevorzugte Emulsion gemäß der vorliegenden Erfindung Diclofenac als NSAID, wobei Diclofenac in einem Konzentrationsbereich von 0,2 - 0,4 Gewichtsprozent der Emulsion enthalten ist. Noch mehr bevorzugt ist es, wenn Diclofenac in einem Konzentrationsbereich von 0,2 - 0,35 Gewichtsprozent der Emulsion vorliegt. Am meisten bevorzugt ist ein Konzentrationsbereich von 0,25 - 0,35 Gewichtsprozent. Höhere Anteile an niederen Alkoholen, wie Ethanol oder Isopropanol, (>10%) verstärken den Reizeffekt und laufen daher den schmerz- und entzündungslindernden Effekten entgegen.

Es ist bekannt, dass die Findung einer Arzneiform zur topischen Applikation in der Scheide dadurch erschwert ist, dass es bei der Anwendung leicht zu einem Auslaufen des Arzneimittels kommen kann, was eine zuverlässige Dosierung erschwert und eine ausreichende Einwirkungsdauer verhindert. In Anbetracht des relativ engen therapeutischen Fensters setzen Medikamente gemäß der vorliegenden Erfindung zur Erzielung des gewünschten therapeutischen Effektes den sicheren Verbleib des Wirkstoffes, vor allem der NSAID-Komponente am Wirkort voraus. Deshalb bewegt sich im Arzneimittel das Verhältnis der Ölphase, welche das Antimykotikum enthält, zur Wasserphase, in welcher sich das NSAID befindet, in einem relativ engen Bereich. Lösungen, Emulsionen oder Gele mit hohem Wasser- und/oder Alkoholgehalt sind als Arzneiformen gemäß der vorliegenden Erfindung auszuschließen, weil ein unkontrollierter Verlust durch Auslaufen aus der Scheide zu erwarten ist.

Halbfeste Emulsionen (Öl in Wasser oder Wasser in Öl), vorzugsweise in Form einer Creme, sind besonders gut geeignet, um die Wirkstoffe effizient und konzentriert an die mit Biofilmen behafteten Oberflächen heranzubringen. Die Viskosität der Emulsionen wird stark durch das Wasser zu Öl Verhältnis bestimmt. Überraschender Weise zeigte sich, dass bei der Formulierung der Emulsion dem Verhältnis Wasser: Öl eine besondere Bedeutung zukommt. Bei höherem Anteil der Fettbestandteile wird die Entfaltung der Wirksamkeit behindert. Hingegen kommt es bei einem niedrigeren Fettanteil zu einer stärkeren Reizwirkung. Zur Erzielung eines optimalen therapeutischen Effektes soll das Wasser: Öl Verhältnis den Wert von 2,7 nicht übersteigen. Über diesem Bereich wird der Wirkstoff zu schnell mit dem Scheidensekret ausgeschwemmt, wodurch keine ausreichende Zeit gegeben ist, um die zur Adhäsionsunterbindung beitragende Wirkung auf die äußere Schicht des Vaginalepithels, an welchem der Pilz haftet, zu entfalten. (Der bei zu hohem Wasser: Öl Verhältnis sehr schnell ausgeschwemmte Wirkstoff kann allenfalls als Nebenwirkung zusätzlich auch einen reizenden Effekt verursachen.)

Es hat sich überraschender Weise gezeigt, dass chronische Pilzerkrankungen der Scheide häufig nicht durch eine stärkere Resistenz der Erreger bedingt sind, sondern vielmehr durch eine zugleich verlaufende zunächst durch die Infektion verursachte chronischen Entzündung des Scheidenepithels. In solchen chronischen Fällen kann eine Heilung nur erzielt werden, wenn der Verbleib des NSAID in der Scheide sichergestellt ist.

In gleicher Weise, wie ein zu hoher Wasseranteil in der Emulsion einen negativen Effekt ausübt, ist auch ein zu hoher Fettanteil zu vermeiden. In Anbetracht der niedrigen Konzentration, in welcher das NSAID eingesetzt wird, ist es zur Erzielung des therapeutischen Effektes von besonderer Wichtigkeit, dass die Freisetzung des Wirkstoffes am Wirkort rasch und nicht protrahiert erfolgt. Bei einer langsamen Freisetzung, wie sie aus der öligen Phase erfolgt, ist nicht gewährleistet, dass die erforderliche therapeutische Konzentration am Wirkort erreicht wird. Aus diesem Grund soll das Wasser: Öl Gewichtsverhältnis in der Emulsion den Wert von 2,0 nicht unterschreiten. Somit ergibt sich auch für den Wert des Wasser-Öl Gewichtsverhältnisses der erfindungsgemäßen Emulsionen ein Fenster zwischen 2,0 und 2,7, vorzugsweise zwischen 2,1 und 2,6, noch mehr bevorzugt zwischen 2,2 und 2,55.

Es ist wesentlich, dass die NSAIDs gemäß der Erfindung in Salzform (bzw. ionischer Form) vorliegen und zwar sowohl bei der Einarbeitung als auch bei der Anwendung. Deshalb ist deren Einbringung in die Formulierung von Bedeutung. Bei einer Einarbeitung des NSAID in seiner freien Form oder bei der Einarbeitung als Salz in die ölige Phase wird der therapeutische Effekt massiv beeinträchtigt. Während das Antimykotikum vorzugsweise in die ölige Phase eingearbeitet wird und in dieser vorliegt, wird daher nach dem Verfahren gemäß der Erfindung das NSAID vor Herstellung der Emulsion in der Regel in die wässrige Phase eingebracht. Alternativ dazu kann das feste Salz des NSAID in feinkristalliner oder mikronisierter Form, bzw. als Hydrogel in die (weitgehend) fertige Emulsion eingearbeitet werden.

Eine zügige Freisetzung ist dann gewährleistet, wenn in der Emulsion der Wirkstoff in der wässrigen Phase vorliegt, was voraussetzt, dass das NSAID als Salz vorliegt. Die meisten NSAIDs sind schwache Säuren mit einem pKa-Wert von 4 - 5 (Diclofenac 4,15, Ibuprofen 4,91, Mefenaminsäure 4,2, Indometacin 4,5, Naproxen 4,2). Demgemäß liegen sie bereits im schwach sauren Milieu zum Teil in freier Form vor und werden so in die Ölphase extrahiert, was zu einer reduzierten Wirkung oder zum Wirkungsverlust führen kann. Da mit sinkendem pH-Wert der Wirkstoffanteil des als freie Säure vorliegenden NSAID in der Ölphase zunimmt, ist ein pH-Wert von mindestens pH = 6 zweckmäßig, um bei den Medikamenten in den Wirkstoffkonzentrationen gemäß der Erfindung einen therapeutischen Effekt zu haben.

Vorzugsweise ist die erfindungsgemäße Emulsion geeignet für die vaginale Applikation. Ungeeignet für die vaginale Applikation sind beispielsweise Zusammensetzungen, die einen hohen Gehalt an schleimhautschädigenden Stoffen, beispielsweise Ethanol oder Isopropanol, aufweisen. Es ist daher bevorzugt, dass die erfindungsgemäße Emulsion nicht mehr als 10 Gew.%, insbesondere nicht mehr als 5 Gew.%, Ethanol enthält. Es ist außerdem bevorzugt, dass die erfindungsgemäße Emulsion nicht mehr als 15 Gew.%, insbesondere nicht mehr als 7,5 Gew.%, Isopropanol enthält.

Die Kombination eines lipophilen Antimykotikums, vom Strukturtyp des Clotrimazol, mit den schwachen Säuren der NSAIDs führt auch dazu, dass die Kombination nur in einem relativ engen pH-Fenster stabil ist. Unter einem pH von 6 wird Clotrimazol instabil, während es bei NSAIDs zu einer Abnahme der chemischen Stabilität im basischen Milieu kommen kann (im Fall von Diclofenac ab pH 8,00 - 8,5). Die chemische Stabilität wird zusätzlich wiederum vom Wasser:Öl-Verhältnis und vom Anteil an alkoholischen Substanzen beeinflusst. In Kombinationsmedikamenten aus Azolen und den sauren NSAIDs entstehen ungünstige Stabilitätsvoraussetzungen aus der direkten Reaktivität der Wirkstoffe. Ebenso muss beachtet werden, dass der pH-Wert einer Emulsion die grundsätzliche physiologische Verträglichkeit beeinflusst. Deshalb sind auch bezüglich des pH-Wertes der Formulierung relativ enge Grenzen gesetzt. Gemäß der Erfindung hat die (wässerige Phase der Emulsion) einen pH-Wert im Bereich von 6,5 bis 8,5, vorzugsweise von 7 - 8.

Da durch die Einbringung des NSAID-Salzes in ein antimykotisches Medikament der pH-Wert alkalischer wird, ist das in vergleichbaren Emulsionen, welche nur Clotrimazol enthalten, üblicherweise verwendete Konservierungsmittel nicht immer ausreichend wirksam. Die halbfesten antimykotischen Medikamente gemäß der vorliegenden Erfindung werden daher vorzugsweise mit solchen Konservierungsmitteln und Antiseptika gegen mikrobiellen Befall geschützt, welche im pH-Bereich der Emulsion wirksam sind. In erfindungsgemäßen Medikamenten, welche zur Behandlung einer reinen Pilzinfektion eingesetzt werden, wird der Gehalt des Antiseptikums zweckmäßiger Weise niedrig gehalten, um die normale Scheidenflora nicht zu beeinträchtigen. In den Biofilmen, welche sich durch das Wachstum der Pilze bilden, sind häufig nicht nur Pilze, sondern auch andere pathogene Mikroorganismen enthalten. Alleine schon durch eine entsprechende Erhöhung der Konzentration des Antiseptikums können auch solche Mischinfektionen therapeutisch erfasst werden. Deshalb ist die Zugabe von einer höheren, für eine akute antimikrobielle Wirkung ausreichenden Menge eines Antiseptikums zu den beanspruchten Medikamenten ein weiterer Gegenstand der Erfindung. Bevorzugte Antiseptika sind quartäre Ammoniumsalze, wie Benzalkoniumchlorid, und Dequaliniumchlorid, sowie Phenoxyethanol. Bevorzugte Konzentration sind mindestens 0,2 Gewichtsprozent für Benzalkoniumchlorid, mindestens 0,2 Gewichtsprozent für Dequaliniumchlorid, und mindestens 2 Gewichtsprozent für Phenoxyethanol.

Unter den bakteriellen Mikroorganismen, welche sich in den vaginalen Biofilmen finden, spielen typische Darmkeime, wie Enterobacter, E. coli und/oder etwa Klebsiella pneumoniae sowie Anaerobier wie Gardnerella vaginalis und Prevotella spp. eine besondere Rolle. Diese werden von den beanspruchten Antiseptika wohl ebenfalls erfasst, es ist jedoch zweckmäßig, in nachgewiesenen Fällen einer derartigen Infektion, an Stelle des Antiseptikums oder ergänzend zu diesem ein gegen Anaerobier aktives Antibiotikum einzusetzen. Deshalb ist die Zugabe eines Antibiotikums, welches gegen anaerobe Keime wirkt, ein weiterer Gegenstand der Erfindung. Bevorzugte Antibiotika sind Phosphomycin, Clindamycin, Metronidazol, Nitrofurantoin, Nitrofurazon, Nitrofurantoin, Nifuratel, Nifuroxacin, Nitroxolin, Trimethoprim, Sulfadiazin und Cotrimoxazol.

Entscheidend für das Aufbrechen des Biofilms ist die Anwesenheit eines NSAID in den beschriebenen Konzentrationen und Bedingungen in einer Emulsion in der beschriebenen Zusammensetzung und in den beschriebenen Mengenverhältnissen.

Schleimhautoberflächen bieten ideale Voraussetzungen für die Ausbildung von Biofilmen, welche besonders therapieresistent sind. Das vaginale Mikrobiom trägt durch die Beeinflussung des feuchten, physiologisch sauren Milieus der Scheide entscheidend zur Wirksamkeit topisch applizierter Arzneiformen bei, da die Verfügbarkeit der Arzneistoffe pHabhängig ist. Diese Bedingungen sind eine der Mitursachen, dass die derzeit vermarkteten topischen Antimykotika nur einen sehr eingeschränkten Erfolg bei der Therapie chronischen Vulvovaginitis, vor allem bei Vorliegen von Mischinfektionen mit Bakterien, haben. Die Zusammensetzung der erfindungsgemäßen Arzneistoffkombinationen ist in den beschriebenen Kombinationen für die Behandlung auch komplexer chronischer Scheidenentzündungen ganz besonders geeignet.

Da es auch bei sehr vielen dermalen Pilzinfektionen zur Ausbildung von Biofilmen kommt, sind Emulsionen gemäß dieser Erfindung auch sehr gut zur Behandlung von Mykosen, insbesondere Candidamykosen, aber auch Infektionen mit Malassezia geeignet.

Demgemäß dienen die erfindungsgemäßen Emulsionen vorzugsweise zur Anwendung in der Behandlung von dermalen und vaginalen Pilzinfektionen, insbesondere zur Anwendung in der topischen Behandlung von vaginalen Pilzinfektionen. Generell können die erfindungsgemäßen Emulsionen zur Anwendung in der Behandlung von Infektionskrankheiten herangezogen werden, insbesondere von vaginalen Infektionen durch Candida albicans oder von vaginalen Mischinfektionen durch Candida albicans und Bakterien, wie Enterobacter, E.coli, Klebsiella pneumoniae, Gardnerella vaginalis, Prevotella spp. Eine besondere Ausführungsform der vorliegenden Erfindung liegt in der Anwendung in der Behandlung bakterieller Vaginosen. Eine weitere besondere Ausführungsform der vorliegenden Erfindung liegt in der Anwendung in der Behandlung dermaler und mukosaler Pilz- und Mischinfektionen vorzugsweise von Candidamykosen, insbesondere vulvovaginale Candidiasis, oropharyngeale Candidiasis (Mundsoor), Windeldermatitis (Windelsoor), Analekzem, intertriginöses Ekzem, und Malasseziamykosen (Pityriasis versicolor).

Da im Falle von chronischen Infektionen die Entzündungen des Epithels weiterbestehen kann, sind auch solche Emulsionen Gegenstand der vorliegenden Erfindung, welche keinen antifungalen oder antimikrobiellen Wirkstoff enthalten. Wegen ihrer verlässlichen Freisetzung des NSAID, welche sich aus der speziellen Zusammensetzung ergibt sind diese sehr gut geeignet, um in der Nachbehandlung von Schleimhautinfektionen, insbesondere von vaginalen Entzündungen eingesetzt zu werden. Solche halbfesten Emulsionen sind auch sehr gut geeignet, um chronische Entzündungen anderer Ursache, besonders Entzündungen von Schleimhäuten und der unmittelbar angrenzenden Gewebe, zu behandeln. Beispiele hierfür ergeben sich in der Nachbehandlung von chronischen Blasenentzündungen, bei atropher Vaginitis oder bei Entzündungen der Analschleimhaut. Die erfindungsgemäßen Emulsionen dienen daher insbesondere zur Anwendung in der Behandlung von Schleimhautentzündungen und Schleimhautinfektionen, insbesondere von vaginalen Infektionen und vaginalen Entzündungen, vor allem chronischen Entzündungen und Infektionen.

Die Erfindung wird anhand der nachfolgenden Figuren und Beispiele näher erläutert, auf die sie jedoch nicht eingeschränkt ist.

### Figuren:

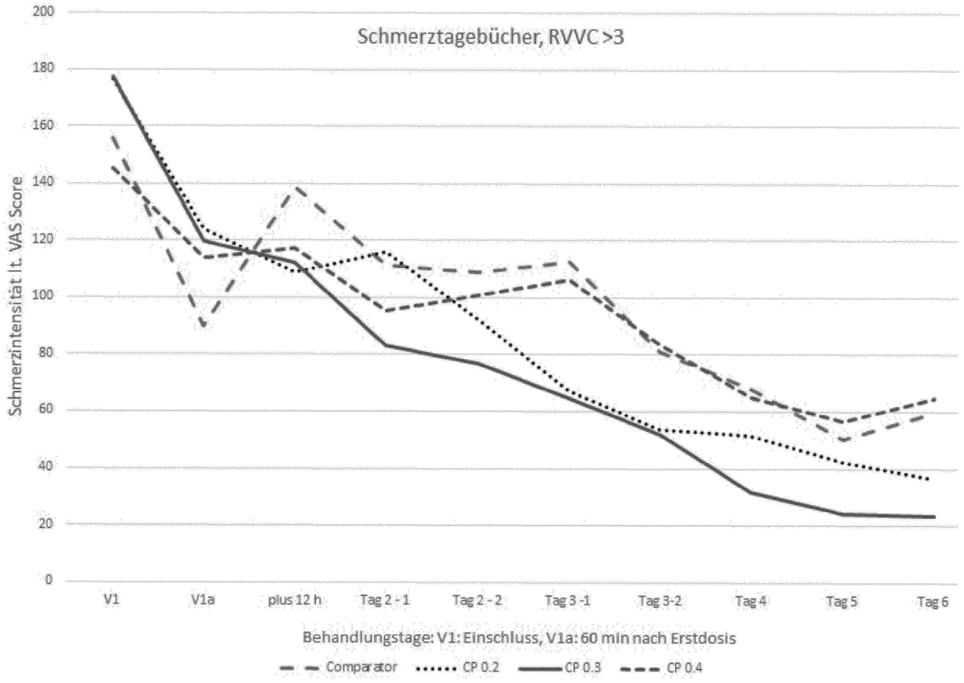

**Figur 1****:** Klinische Phase II Studie. Schmerztagebücher von Patientinnen mit rezidivierender vulvovaginaler Candidiasis (RVVC), die mit erfindungsgemäßen Emulsionen enthaltend unterschiedliche Diclofenac-Na Konzentrationen (CP1: 0,2 Gew%, CP2: 0,3 Gew%, CP3: 0,4 Gew%) oder mit einer Kontrollzusammensetzung ohne Diclofenac-Na ("Comparator") behandelt wurden. Die Schmerzintensität wurde von den Patientinnen zu den Erfassungszeitpunkten in einer Schmerzskala von 0 bis 10 eingetragen. Das Diagramm zeigt die Mittelwerte der Behandlungsgruppen im Verlauf der Behandlung. Bei CP 1 ist eine rasche Abnahme der Schmerzintensität gegeben, Die Heilungskurve flacht allerdings ab, sobald die Tagesdosis halbiert wird (ab Tag 4). CP 2 zeigt einen optimalen Heilungsverlauf. Bereits am 2.Tag ist das Schmerzniveau auf 50% des Ausgangswertes gesunken.

### Beispiele:

### Vorbemerkung

Veränderungen im Herstellungsverfahren und in der prozentuellen Zusammensetzung eines Öl-Wassergemisches entsprechend Basisrezeptur A zeigten überraschende Veränderungen der klinischen Wirksamkeit abseits von üblichen Dosis-Wirkungsbeziehungen. Ableitbar ist die deutlich verbesserte oder ggf. auch verringerte Wirksamkeit aus einem besonders raschen Wirkungseintritt (lokale Schmerzstillung) bzw. einer Verzögerung des Wirkungseintrittes oder der Verstärkung vorbestehender Schmerzen.

### Beispiel 1 - Basisrezeptur

**Tab.1: Basisrezeptur A**

| Zusammensetzung der Emulsion | | |
|---|---|---|
| Clotrimazol | | 1,00 |
| Diclofenac Na | | 0,20 |
| Sorbitanmonostearat | | 2,00 |
| Polysorbat 60 | | 1,50 |
| Cetylpalmitat | | 3,00 |
| 2-Octyldodecanol | | 13,50 |
| Cetostearylalkohol | | 10,00 |
| Benzylaalkohol | | 1,00 |
| Gereinigtes Wasser Ph.Eu. | | 67,80 |
| Gesamt | | 100,00 |
| Ph | | 7,8 |

Bei Konzentrationsänderungen des NSAIDs es wird dieses jeweils gegen gereinigtes Wasser ausgetauscht, der Gehalt der Lipidbestandteile bleibt, wenn nicht anders angeführt, gleich. Die klinische Wirksamkeit in den folgenden angeführten Präparationen wird in Bezug zu einer klinisch gut wirksamen Basisrezeptur A gesetzt.

### Beispiel 2: Variationen des pH-Bereiches und klinische Wirksamkeit

Veränderungen in Bezug auf die Konservierungsmittel sind mit Veränderungen des pH-Wertes verbunden. Die Herstellung der genannten Beispiele erfolgt nach allgemeiner Arbeitsvorschrift 1. Die Einstellung des pH-Wertes zur Erreichung des Wirkungsoptimums des jeweiligen Konservierungsmittels erfolgt durch Zugabe geeigneter Pufferlösungen, durch welche die jeweilige Menge an gereinigtem Wasser ersetzt wurde.

Der pH-Wert hat über die Verschiebung des freien Wirkstoffanteils gegenüber dem als Salz vorliegenden Anteil einen wesentlichen Einfluss auf die lokal bioverfügbare Wirkstoffmenge. In Abhängigkeit von den pKa-Werten der eingesetzten nicht-steroidalen Antiphlogistika ergibt sich daraus ein pH-Optimum der erfindungsgemäßen Kombinationspräparate gemäß der vorliegenden Erfindung. Die Zusammensetzung mit einem pH-Wert von 5,6 ist ein Referenzbeispiel.

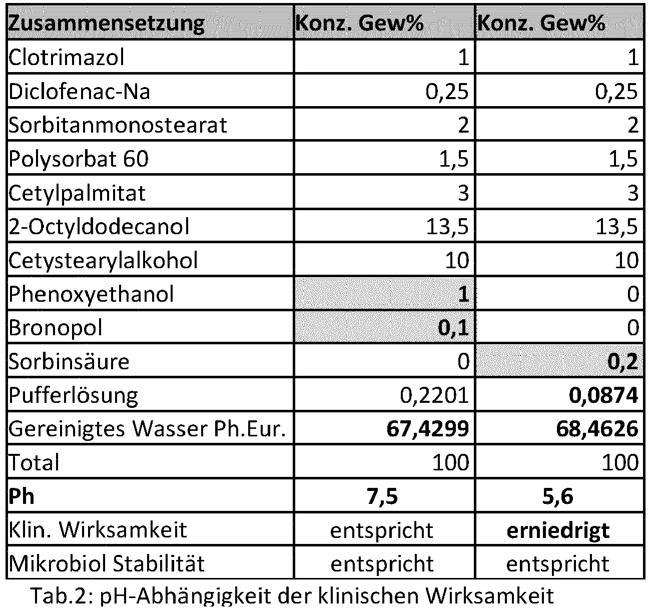

### Beispiel 3: Einfluss des Gewichtsverhältnisses wässerige Phase/Öl

Veränderungen der Viskosität zeigen überraschenderweise schon bei geringer Variationsbreite deutliche Einflüsse auf die klinische Wirksamkeit. Die Herstellung der genannten Beispiele erfolgt nach allgemeiner Arbeitsvorschrift 1 durch Variationen im Gehalt der fettigen Bestandteile und des Wassergehaltes.

Eine Erhöhung des Wasseranteils und damit Erniedrigung der Viskosität führt über eine verstärkte Freisetzung und stärkere Benetzung der Schleimhäute zu lokalen Reizerscheinungen und verminderter klinischer Wirksamkeit.

**Tab.3: Einfluss des Gewichtsverhältnisses wässerige Phase/Öl auf die klinische Wirksamkeit**

| | **Phase** | **Fettanteil / Viskosität erniedrigt** | | **Basisformulierungen** | | **Fettanteil** / **Viskosität erhöht** | |
|---|---|---|---|---|---|---|---|
| Clotrimazol | Öl | 1 | 1,0 | 1,0 | 1,0 | 1,0 | 1,0 |
| Diclofenac-Na | Wasser | 0,3 | 0,3 | 0,3 | 0,3 | 0,3 | 0,3 |
| Sorbitanmonostearat | - | 2,0 | 2,0 | 2,0 | 2,0 | 2,0 | 2,0 |
| Polysorbat 60 | - | 1,5 | 1,5 | 1,5 | 1,5 | 1,5 | 1,5 |
| Cetylpalmitat | Öl | 3,0 | 3,0 | 3,0 | 3,0 | 3,0 | 3,0 |
| 2-Octyldodecanol | Öl | 13,5 | 13,5 | 13,5 | 13,5 | 13,5 | 14,5 |
| Cetystearylalkohol | Öl | 7,5 | 5 | 10 | 10 | 14 | 16 |
| Benzylalkohol | Öl | 1,0 | 1,0 | 1,0 | | 1,0 | 1,0 |
| Phenoxyethanol | Öl | | | | 1,0 | | |
| Propylenglykol | Wasser | | | | 7 | | |
| Wasser | Wasser | 70,2 | 72,7 | 67,75 | 60,7 | 63,7 | 60,7 |
| Total | | 100 | 100 | 100 | 100 | 100 | 100 |
| Klinische Wirksamkeit | | **reizend** | **reizend** | **entspricht** | **entsprich t** | **erniedrigt** | **erniedrigt** |
| | | | | | | | |
| Wasserphase gesamt | | 70,5 | 73,0 | 68,0 | 68,0 | 64,0 | 61,0 |
| Fettphase gesamt | | 26 | 23,5 | 28,5 | 28,5 | 32,5 | 35,5 |
| **wässrige Phase / Fett** | | **2,7** | **3,1** | **2,4** | **2,4** | **2,0** | **1,7** |

Formulierungen mit einem Verhältnis von wässriger zu Ölphase außerhalb 2,0 bis 2,7 sind Referenzformulierungen.

Zur Berechnung des Gewichtsverhältnisses der Wasser- zur Ölphase werden die einzelnen Anteile der Wasser- und der Ölphase wie in der Tabelle gezeigt aufsummiert. Da Emulgatoren, z.B. Sorbitanmonostearat und Polysorbat 60, an den Grenzflächen zwischen den zwei Phasen liegen, sind sie weder der Wasser- noch der Ölphase zuzurechnen.

Berechnet man das Verhältnis der wässrigen Phase zur Ölphase der konzentrierten Emulsion (d.h. dem Zwischenprodukt aus Komponenten A, B, J, C, E, G, H und der Hälfte von K) von Beispiel 2 der WO 02/0768648 A2, erhält man ein Verhältnis von 3,1 (Ölphase: Terbinafin, Butylhydroxytoluol, Benzylalkohol, Isopropylmyristat, gesamt 11,52 g / 100 g; Wasserphase: Diclofenac-Natrium und Wassers, gesamt 35,94 g / 100 g; Verhältnis 3,1). Eine derartige Emulsion hat daher ein Wasser:Öl Verhältnis außerhalb des erfindungsgemäßen Bereichs und wäre daher im Zusammenhang mit der vorliegenden Erfindung nicht geeignet.

Alternativ könnte das Gewichtsverhältnis der Wasser- zur Ölphase ohne Einbeziehen der in den Phasen gelösten Stoffen (Clotrimazol, Diclofenac-Na, Benzylalkohol, Cetylstearylalkohol) berechnet werden. Bei dieser Berechnungsweise würde in Tab. 3 lediglich Wasser und Propylenglykol der Wasserphase, und Cetylpalmitat, 2-Octyldodecanol und Cetystearylalkohol der Ölphase zugerechnet werden. Die in Tab. 3 angegebenen Wasser-Öl Verhältnisse 2,7, 3,1, 2,4, 2,4, 2,0, 1,7 würden gemäß dieser Berechnungsweise den Werten 2,9, 3,4, 2,6, 2,6, 2,1, 1,8 entsprechen. Der erfindungsgemäße Bereich von 2,0 bis 2,7 würde bei dieser Berechnungsweise einem Bereich von 2,1 bis 2,9 entsprechen.

Im Rahmen der vorliegenden Erfindung soll die Berechnung des Gewichtsverhältnisses der Wasser- zur Ölphase aber wie in Tab. 3 gezeigt erfolgen, d.h. unter Einbeziehen der in den Phasen gelösten Stoffen.

### Beispiel 4: Variation des nicht-steroidalen Antiphlogistikums

Zu Basisrezeptur A wurden anstelle von Diclofenac verschiedene andere nicht-steroidale Antiphlogistika beigemengt und auf ihre klinische Wirksamkeit untersucht.

**Tab.4: Variationen von nicht-steroidalen Antiphlogistika**

| Formu lierung Nr. | Nicht-steroidiales Antiphlogistikum | Präparation | Herstellungsvariante | Wirksamkeit |
|---|---|---|---|---|
| 1.1 | Mefenaminsäure | 1 g/100 g | Basisrezeptur A + Mefenaminsäure mikronisiert | entspricht |
| 1.2 | Indometacin | 0,15 g/100 g | Basisrezeptur A + Indometacin mikronisiert | entspricht |
| 1.3 | Ibuprofen | 1 g/100 g | Basisrezeptur A + Ibuprofen in Hydrogel | entspricht |
| 1.4 | Ibuprofen | 0,5 g/100 g | Basisrezeptur A + Ibuprofen in Hydrogel | entspricht |
| 1.5. | Naproxen | 1 g/100 g | Basisrezeptur A + Naproxen mikronisiert | entspricht |

### Beispiel 5: Konzentrationsoptimum

Gemäß Basisrezeptur A wurden Emulsionen mit verschiedenen Konzentrationen von Diclofenac Na hergestellt und auf ihre klinische Wirksamkeit untersucht.

**Tab.5: Abhängigkeit der klinischen Wirksamkeit von der Konzentration des NSAIDs**

| | **Konz. Gew%** | **Konz. Gew%** | **Konz. Gew%** | **Konz. Gew%** |
|---|---|---|---|---|
| Clotrimazol | 1 | 1 | 1 | 1 |
| Diclofenac-Na | 0,1 | 0,25 | 0,5 | 0,75 |
| Sorbitanmonostearat | 2 | 2 | 2 | 2 |
| Polysorbat 60 | 1,5 | 1,5 | 1,5 | 1,5 |
| Cetylpalmitat | 3 | 3 | 3 | 3 |
| 2-Octyldodecanol | 13,5 | 13,5 | 13,5 | 13,5 |
| Cetystearylalkohol | 10 | 10 | 10 | 10 |
| Benzylalkohol | 1 | 1 | 1 | 1 |
| Gereinigtes Wasser Ph.Eur. | 67,9 | 67,75 | 67,5 | 67,25 |
| Total | 100 | 100 | 100 | 100 |
| **Ph** | **7,6** | **7,8** | **8,1** | **8,1** |
| Klin. Wirksamkeit | leicht erniedrigt | entspricht | entspricht, leicht reizend | schleimhautreizend |

### Beispiel 6: Variation von Konservierungsmitteln und mikrobiologische Stabilität

Durch den gemeinsamen Einsatz von Clotrimazol und NSAIDs verändern sich durch die pH-Verschiebungen im System der Emulsion sowohl mikrobiologische als auch die chemische Stabilität [Lit. Pharmakopöe] gegenüber einer vergleichbaren Clotrimazolformulierung.

Die Formulierung mit einem pH-Wert von 5,6 ist eine Referenzformulierung.

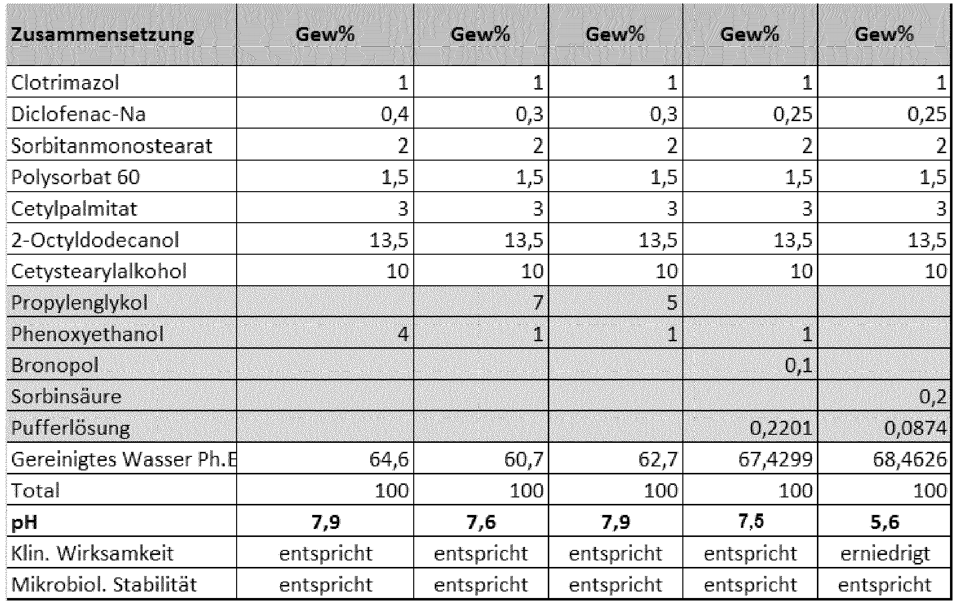

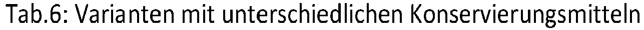

### Beispiel 7:

### Herstellung von Basisrezeptur A,

### Allgemeine Herstellungsvorschrift 1:

Die Komponenten Sorbitanmonostearat, Polysorbat 60, Cetylpalmitat, 2-Octyldodecanol und Cetostearylalkohol werden bei einer Temperatur von 70-75°C aufgeschmolzen. In die Klarschmelze werden unter Rühren bei einer Temperatur von 60 °C - 70 °C Clotrimazol und anschließend Benzylalkohol zugegeben. Parallel hierzu wird Diclofenac Natrium in gereinigtem Wasser unter Erwärmung aufgelöst. Die wässrige Lösung wird unter Rühren zur Ölphase zugegeben und homogenisiert. Unter langsamem Abkühlen unter weiterer Homogenisierung der entstehenden w/o-Emulsion findet eine Phasenumkehr statt, bei welcher eine hydrophile, homogene Creme entsteht.

**Tab. 8: Formulierungen nach Basis Rezeptur A, Herstellungsvorschrift 1**

| Bestandteile der Emulsion | | | |
|---|---|---|---|
| Clotrimazol | | 1,00 | 1,00 |
| Diclofenac Na | | 0,20 | 0,30 |
| Sorbitanmonostearat | | 2,00 | 2,00 |
| Polysorbat 60 | | 1,50 | 1,50 |
| Cetylpalmitat | | 3,00 | 3,00 |
| 2-Octyldodecanol | | 13,50 | 13,50 |
| Cetostearylalkohol | | 10,00 | 10,00 |
| Benzylalkohol | | 1,00 | 1,00 |
| Gereinigtes Wasser Ph.Eu. | | 67,80 | 67,70 |
| | | 100,00 | 100,00 |

Die klinische Wirksamkeit der Rezepturen in Tabelle 8 ist ident.

### Allgemeine Herstellungsvorschrift 2

Die Komponenten Sorbitanmonostearat, Polysorbat 60, Cetylpalmitat, 2-Octyldodecanol und Cetostearylalkohol werden bei einer Temperatur von 70-75°C aufgeschmolzen. In die Klarschmelze wird unter Rühren bei einer Temperatur von 60 °C - 70 °C Clotrimazol zugegeben und aufgeschmolzen. Parallel hierzu wird Diclofenac Natrium in gereinigtem Wasser unter Erwärmung aufgelöst. Nach Zugabe von Phenoxyethanol und gegebenenfalls Propylenglykol wird die wässrige Lösung bei einer Temperatur von 60°C - 70°C C unter Rühren zur Ölphase zugegeben und homogenisiert. Unter langsamem Abkühlen unter weiterer Homogenisierung der anfangs entstehenden w/o-Emulsion findet eine Phasenumkehr zu einer o/w-Emulsion statt, bei welcher eine hydrophile, homogene Creme entsteht. (Klin. Ergebnisse siehe Tab.6)

Die Einarbeitung des NSAIDs kann anstelle des Auflösens in Wasser auch durch Einrühren eines in einem Hydrogel aufgelösten NSAID oder durch Einrühren des (mikronisierten) NSAIDs als Feststoff während der Emulsionsbereitung erfolgen.

### Beispiel 9: Herstellung von Basisrezeptur A durch Einarbeitung des NSAID in die Ölphase

Cremes mit verschiedenen Konzentrationen von Diclofenac Na wurden nach Basisrezeptur A nach allgemeiner Herstellungsvorschrift 2 hergestellt und auf ihre klinische Wirksamkeit untersucht. Gereinigtes Wasser wurde gegen Diclofenac Na ausgetauscht, der Gehalt der Lipidbestandteile, des O/W-Emulgators Polysorbat 60 und des Konservierungsmittels Benzylalkohol war in den Rezepturen identisch.

### Allgemeine Herstellungsvorschrift 3

Die Komponenten Sorbitanmonostearat, Polysorbat 60, Cetylpalmitat, 2-Octyldodecanol und Cetostearylalkohol werden aufgeschmolzen. In die Klarschmelze wird unter Rühren Benzylalkohol gegeben (Lipidphase). Gereinigtes Wasser wird zum Sieden erhitzt und 70% der erforderlichen Masse entsprechend Rezeptur unter Rühren in die Lipidphase gegeben (Phasenumkehr und Bildung einer O/W-Präemulsion). Etwa 30% der Präemulsion werden aus dem Kessel genommen und darin Clotrimazol und Diclofenac-Natrium dispergiert. Die wirkstoffhaltige Präemulsion wird in den Kessel zur wirkstofffreien Präemulsion gegeben und mit gereinigtem Wasser (restliche 30%) auf die Endmasse aufgefüllt. Die Creme wird bis zum Erreichen von Raumtemperatur gerührt und abschließend zur Homogenisierung zweimal über den Dreiwalzenstuhl gegeben (pH 6,99).

**Tab. 9: Formulierungen nach Basis Rezeptur A, Herstellungsvorschrift 3.**

| Zusammensetzung | | | |
|---|---|---|---|
| Clotrimazol | | 2,00 | 2,00 |
| Diclofenac Na | | 0,10 | 0,20 |
| Sorbitanmonostearat | | 2,00 | 2,00 |
| Polysorbat 60 | | 1,50 | 1,50 |
| Cetylpalmitat | | 3,00 | 3,00 |
| 2-Octyldodecanol | | 13,50 | 13,50 |
| Cetostearylalkohol | | 10,00 | 10,00 |
| Benzylaalkohol | | 1,00 | 1,00 |
| Gereinigtes Wasser Ph.Eu. | | 66,90 | 66,80 |
| | | 100,00 | 100,00 |
| Klin. Wirksamkeit | | erniedrigt | erniedrigt |

Aus den Beispielen nach Herstellungsvorschrift 3 ergibt sich ein wesentlicher Einfluss des Herstellungsprozesses, der so gestaltet sein muss, dass das nicht-steroidale Antiphlogistikum über die wässrige Phase eingearbeitet wird und vorwiegend in der wässrigen Phase vorliegt.

### Beispiel 10: Herstellung von Basisrezeptur A

Herstellung von Basisrezeptur A mit Einarbeitung eines nicht-steroidialen Antiphlogistikums (Diclofenac Na) nach allgemeiner Herstellungsvorschrift 4:
Die Komponenten Sorbitanmonostearat, Polysorbat 60, Cetylpalmitat, 2-Octyldodecanol und Cetostearylalkohol werden aufgeschmolzen. In die Klarschmelze wird unter Rühren Benzylalkohol gegeben (Lipidphase). Gereinigtes Wasser wird zum Sieden erhitzt und 70% der erforderlichen Masse entsprechend Rezeptur unter Rühren in die Lipidphase gegeben (Phasenumkehr und Bildung einer O/W-Präemulsion). Etwa 30% der Präemulsion werden aus dem Kessel genommen und darin Clotrimazol dispergiert. Die wirkstoffhaltige Präemulsion wird in den Kessel zur wirkstofffreien Präemulsion gegeben und mit gereinigtem Wasser (restliche 30%) auf die Endmasse aufgefüllt. Die Creme wird unter Zugabe von mikronisiertem Diclofenac Natrium in mehreren Portionen bis zum Erreichen der Raumtemperatur gerührt und abschließend zur Homogenisierung zweimal über den Dreiwalzenstuhl gegeben.

### Beispiel 11: Klinische Phase II Studie

Die klinische Wirksamkeit von erfindungsgemäßen Emulsionen zur Behandlung von chronischen Vaginalinfektionen wurde in einer klinischen Phase II Studie untersucht.

Gemäß Allgemeiner Herstellungsvorschrift 1 wurden drei erfindungsgemäße Emulsionen mit verschiedenen Konzentrationen von Diclofenac Na hergestellt. Als Kontrolle wurde eine Emulsion ohne NSAID verwendet:
- Emulsion CP1: 0,2 Gew.% Diclofenac Na
- Emulsion CP2: 0,3 Gew.% Diclofenac Na
- Emulsion CP3: 0,4 Gew.% Diclofenac Na
- Emulsion Comparator: 0 Gew.% Diclofenac Na

Behandelt wurden 31 Patientinnen mit chronisch-rezidivierenden Vaginalinfektionen (chronischrezidivierende vulvovaginale Candidiasis, RVVC). Bei Vorliegen einer entsprechenden klinischen Symptomatik mit mindestens mittelgradigen Beschwerden wurden die Patientinnen nach positivem Pilznachweis in die Studie eingeschlossen. In den ersten 3 Tagen wurden zweimal täglich 2,5 ml Creme mittels Applikator intravaginal verabreicht, in den folgenden 3 Tagen wurde einmal täglich 2,5 ml Creme appliziert. Nach 7 - 10 Tagen erfolgte eine Kontrolluntersuchung.

Jede der drei erfindungsgemäßen Emulsionen führte zu einem besseren Heilerfolg als die Kontrolle, die kein NSAID enthielt. Als besonders vorteilhaft erwies sich Emulsion 2, enthaltend 0,3 Gew% Diclofenac-Na. Eine komplette klinische Heilung (komplette Beschwerdefreiheit bei Kontrolluntersuchung durch den behandelnden Arzt) wurde bei allen Patientinnen dieser Gruppe erzielt. In der Kontrollgruppe wurde lediglich eine Heilung bei 40% der Patientinnen erreicht.

Als weiterer klinischer Parameter - zusätzlich zum Heileffekt - wurde die Entwicklung der Schmerzen bei den chronischen Patientinnen (Schmerztagebuch) herangezogen. Die Schmerzintensität wurde von den Patientinnen zu den Erfassungszeitpunkten in einer Schmerzskala von 0 bis 10 eingetragen. Die Mittelwerte der Behandlungsgruppen im Verlauf der Behandlung sind in Figur 1 dargestellt. Bei gleichbleibender Konzentration des Antimykotikums ergab sich, dass in der höchsten Konzentration des NSAIDs (Emulsion 3) der Heilverlauf zwar einen besseren Verlauf im Vergleich zur Kontrolle ergab, die Abnahme der Schmerzintensität jedoch keinen wesentlichen Unterschied zur Kontrollgruppe zeigte. Die optimale Konzentration (Emulsion 2), bei welcher alle Patientinnen geheilt waren, führte rasch zur vollständigen Reduktion der Schmerzen. Die deutliche Dosisabhängigkeit zeigte sich auch bei den Patientinnen mit der niedrigsten Konzentration (Emulsion 1), bei welchen die Schmerzen deutlich abnahmen, bis von deiner zweimaltägigen auf eine einmaltägige Dosierung umgestellt wurde, worauf sich der Heilungsprozess entsprechend verlangsamte (siehe Figur 1).

In parallel durchgeführten mikrobiologischen Tests konnte gezeigt werden, dass keine der Patientinnen mit resistenten Candida-Stämmen infiziert war, dennoch sprach der überwiegende Teil der RVVC-Patientinnen nicht auf die antifungale Therapie an.

## Patentansprüche

1. Eine Emulsion mit einer wässerigen Phase und einer Ölphase, enthaltend ein Antimykotikum und ein NSAID, **dadurch gekennzeichnet, dass** (a) das NSAID Diclofenac in einer Konzentration von 0,1 bis 0,5 Gewichtsprozent, Indometacin in einer Konzentration von 0,1 bis 0,4 Gewichtsprozent, Naproxen in einer Konzentration von 1 bis 5 Gewichtsprozent, Ibuprofen in einer Konzentration von 0,5 bis 2,5 Gewichtsprozent, Dexibuprofen in einer Konzentration von 0,25 bis 1,25 Gewichtsprozent, Ketoprofen in einer Konzentration von 0,25 bis 1,25 Gewichtsprozent, Mefenaminsäure in einer Konzentration von 0,5 bis 4 Gewichtsprozent, oder Lornixocam in einer Konzentration von 0,02 bis 0,04 Gewichtsprozent ist, wobei das NSAID in Salzform vorliegt; dass (b) das Gewichtsverhältnis der Wasser- zur Ölphase in dieser Emulsion zwischen den Werten 2,0 und 2,7 liegt, wobei das Gewichtsverhältnis unter Einbeziehen der in den Phasen gelösten Stoffen berechnet wird, wobei Emulgatoren weder der Wasser- noch der Ölphase zugerechnet werden, und dass (c) der pH Wert der Emulsion nicht unter dem Wert 6,5 und nicht über 8,5 vorzugsweise im Bereich 7,0 bis 8,0 liegt.

2. Eine Emulsion gemäß Anspruch 1 **dadurch gekennzeichnet, dass** sie in Form einer Salbe oder einer Creme vorliegt.

3. Eine Emulsion gemäß Anspruch 1 oder 2, **dadurch gekennzeichnet, dass** die Emulsion halbfest ist.

4. Eine Emulsion gemäß einem der Ansprüche 1 bis 3, **dadurch gekennzeichnet, dass** das Antimykotikum Nystatin, Ciclopirox oder Ciclopiroxolamin, oder ein Antimykotikum aus der Gruppe der Azole, vorzugsweise Clotrimazol, Fluconazol, Miconazol, Itraconazol, Tioconazol, Voriconazol, Bifonazol, Econazol, Isoconazol, Fenticonazol, Sertaconazol, Ketoconazol, Posaconazol, Quilseconazol, Oteseconazol (VT-1161) oder Ibrexafungerp (SCY-078), insbesondere Clotrimazol, ist.

5. Eine Emulsion gemäß einem der Ansprüche 1 bis 4, **dadurch gekennzeichnet, dass** das NSAID Diclofenac ist und dieses in einem Konzentrationsbereich von 0,2 - 0,4 Gewichtsprozent der Emulsion enthalten ist.

6. Eine Emulsion gemäß einem der Ansprüche 1 bis 5, in welcher ein im pH-Bereich der Emulsion aktives Konservierungsmittel enthalten ist, vorzugsweise wobei das Konservierungsmittel Phenoxyethanol oder Propylenglykol oder eine Kombination dieser beiden, oder Dequaliniumchlorid ist.

7. Eine Emulsion gemäß einem der Ansprüche 1 bis 6, **dadurch gekennzeichnet, dass** weiters ein gegen bakterielle Keime wirkendes Antibiotikum enthalten ist, wobei das das Antibiotikum vorzugsweise Phosphomycin, Clindamycin, Metronidazol, Nitrofurantoin, Nitrofurazon, Nitrofurantoin, Nifuratel, Nifuroxacin, Nitroxolin, Trimethoprim, Sulfadiazin, oder Cotrimoxazol ist.

8. Eine Emulsion gemäß einer der Ansprüche 1 bis 7, **dadurch gekennzeichnet, dass** weiters ein Antiseptikum enthalten ist, vorzugsweise in einer für eine akute antimikrobielle Wirkung ausreichenden Menge, wobei das Antiseptikum vorzugsweise ein quartäres Ammoniumsalz ist, vorzugsweise ausgewählt aus der Gruppe bestehend aus: Benzalkoniumchlorid, vorzugsweise in einer Konzentration von mindestens 0,2 Gewichtsprozent; Dequaliniumchlorid, vorzugsweise in einer Konzentration von mindestens 0,2 Gewichtsprozent; und Phenoxyethanol, vorzugsweise in einer Konzentration von mindestens 2 Gewichtsprozent.

9. Emulsion gemäß einem der Ansprüche 1 bis 8 zur Anwendung in der Behandlung von vaginalen Pilzinfektionen, insbesondere von chronischen vaginalen Pilzinfektionen, vorzugsweise wobei die Behandlung eine topische Behandlung ist.

10. Emulsion zur Anwendung gemäß Anspruch 9, **dadurch gekennzeichnet, dass** die vaginale Pilzinfektion eine vaginale Mischinfektion durch Candida albicans und Bakterien, wie Enterobacter, E.coli, Klebsiella pneumoniae, Gardnerella vaginalis, Prevotella spp. ist.

11. Emulsion zur Anwendung gemäß Anspruch 9 oder 10, **dadurch gekennzeichnet, dass** die vaginale Pilzinfektion eine Candidamykose ist, insbesondere vulvovaginale Candidiasis.

12. Ein Verfahren zur Herstellung einer Emulsion gemäß einem der Ansprüche 1 bis 8, **dadurch gekennzeichnet, dass** bei der Herstellung der Emulsion das NSAID über die wässrige Phase eingebracht wird.

13. Ein Verfahren zur Herstellung einer Emulsion gemäß einem der Ansprüche 1 bis 8, **dadurch gekennzeichnet, dass** das NSAID als feinkristallines oder mikronisiertes Salz in die das Antimykotikum enthaltene Emulsion eingebracht wird.

14. Ein Verfahren zur Herstellung einer Emulsion gemäß einem der Ansprüche 1 bis 8, **dadurch gekennzeichnet, dass** das NSAID über ein Hydrogel in die das Antimykotikum enthaltene Emulsion eingebracht wird.

15. Ein Verfahren zur Herstellung einer Emulsion gemäß Anspruch 7 oder 8, bei welchem die Zugabe der Stoffe so erfolgt, dass eine therapeutisch wirksame antibakterielle Wirkung erzielt wird.

## Claims

1. An emulsion having an aqueous phase and an oil phase containing an antifungal agent and an NSAID, **characterized in that** (a) the NSAID is diclofenac in a concentration of 0.1 to 0.5 weight percent, indometacin in a concentration of 0.1 to 0.4 weight percent, naproxen in a concentration of 1 to 5 weight percent, ibuprofen in a concentration of 0, 5 to 2.5 weight percent, dexibuprofen in a concentration of 0.25 to 1.25 weight percent, ketoprofen in a concentration of 0.25 to 1.25 weight percent, mefenamic acid in a concentration of 0.5 to 4 weight percent, or lornixocam in a concentration of 0.02 to 0.04 weight percent, wherein the NSAID is in salt form; **in that** (b) the weight ratio of the water phase to the oil phase in said emulsion is between the values 2.0 and 2.7, wherein the weight ratio is calculated including the fabrics dissolved in the phases, wherein emulsifiers are not included in either the water phase or the oil phase, and **in that** (c) the pH of the emulsion is not less than the value 6.5 and not more than 8.5, preferably in the area 7.0 to 8.0.

2. An emulsion according to claim 1 **characterized in that** it is in the form of an ointment or a cream.

3. An emulsion according to claim 1 or 2, **characterized in that** the emulsion is semi-solid.

4. An emulsion according to any of claims 1 to 3, **characterized in that** the antifungal agent is nystatin, ciclopirox or ciclopiroxolamine, or an antifungal agent selected from the group of azoles, preferably clotrimazole, fluconazole, miconazole, itraconazole, tioconazole, voriconazole, bifonazole, econazole, isoconazole, fenticonazole, sertaconazole, ketoconazole, posaconazole, quilseconazole, oteseconazole (VT-1 161) or ibrexafungerp (SCY-078), in particular clotrimazole.

5. An emulsion according to any of claims 1 to 4, **characterized in that** the NSAID is diclofenac and this is contained in a concentration range of 0.2- 0.4 weight percent of the emulsion.

6. An emulsion according to any of claims 1 to 5, wherein a preservative active in the pH area of the emulsion is contained, preferably wherein the preservative is phenoxyethanol or propylene glycol or a combination thereof, or dequalinium chloride.

7. An emulsion according to any of claims 1 to 6, **characterized in that** further an antibiotic active against bacterial germs is contained, wherein said antibiotic is preferably phosphomycin, clindamycin, metronidazole, nitrofurantoin, nitrofurazone, nitrofurantoin, nifuratel, nifuroxacin, nitroxolin, trimethoprim, sulfadiazine, or cotrimoxazole.

8. An emulsion according to any of claims 1 to 7, **characterized in that** an antiseptic is further contained, preferably in an amount sufficient for acute antimicrobial activity, wherein the antiseptic is preferably a quaternary ammonium salt, preferably selected from the group consisting of: benzalkonium chloride, preferably in a concentration of at least 0.2 weight percent; dequalinium chloride, preferably in a concentration of at least 0.2 weight percent; and phenoxyethanol, preferably in a concentration of at least 2 weight percent.

9. The emulsion according to any of claims 1 to 8 for use in treating vaginal fungal infections, particularly chronic vaginal fungal infections, preferably wherein the treatment is a topical treatment.

10. The emulsion for use according to claim 9, **characterized in that** the vaginal fungal infection is a mixed vaginal infection by Candida albicans and bacteria such as Enterobacter, E.coli, Klebsiella pneumoniae, Gardnerella vaginalis, Prevotella spp.

11. The emulsion for use according to claim 9 or 10, **characterized in that** the vaginal fungal infection is a candidamycosis, in particular vulvovaginal candidiasis.

12. A method for the production of an emulsion according to any of claims 1 to 8, **characterized in that** in the production of the emulsion the NSAID is introduced via the aqueous phase.

13. A method for the production of an emulsion according to any of claims 1 to 8, **characterized in that** the NSAID is incorporated as a fine crystalline or micronized salt into the emulsion containing the antifungal agent.

14. A method for the production of an emulsion according to any of claims 1 to 8, **characterized in that** the NSAID is introduced into the emulsion containing the antifungal agent via a hydrogel.

15. A method for the production of an emulsion according to claim 7 or 8, wherein the addition of the fabrics is such as to provide a therapeutically effective antibacterial effect.

## Revendications

1. Emulsion avec une phase aqueuse et une phase huileuse, contenant un antimycosique et un AINS, **caractérisée en ce que** (a) l'AINS est le diclofénac à une concentration de 0,1 à 0,5 % en poids, l'indométhacine à une concentration de 0,1 à 0,4 % en poids, le naproxène à une concentration de 1 à 5 % en poids, l'ibuprofène à une concentration de 0,5 à 2,5 % en poids, le dexibuprofène à une concentration de 0,25 à 1,25 % en poids, le kétoprofène à une concentration de 0,25 à 1,25 % en poids, l'acide méfénamique à une concentration de 0,5 à 4 % en poids ou le lornixocam à une concentration de 0,02 à 0,04 % en poids, où l'AINS est sous forme de sel; **en ce que** (b) le rapport pondéral de la phase aqueuse à la phase huileuse dans cette émulsion est situé entre les valeurs 2,0 et 2,7, où le rapport pondéral est calculé en tenant compte des substances dissoutes dans les phases, où des émulsifiants ne sont comptés ni dans la phase aqueuse ni dans la phase huileuse, et **en ce que** (c) le pH de l'émulsion n'est pas inférieur à la valeur 6,5 et pas supérieur à 8,5, de préférence dans la gamme de 7,0 à 8,0.

2. Emulsion selon la revendication 1 **caractérisée en ce qu'**elle est sous la forme d'une pommade ou d'une crème.

3. Emulsion selon la revendication 1 ou 2 **caractérisée en ce que** l'émulsion est semi-solide.

4. Emulsion selon l'une des revendications 1 à 3 **caractérisée en ce que** l'antimycosique est la nystatine, le ciclopirox ou la ciclopiroxolamine, ou un antimycosique du groupe des azoles, de préférence le clotrimazole, le fluconazole, le miconazole, l'itraconazole, le tioconazole, le voriconazole, le bifonazole, l'éconazole, l'isoconazole, le fenticonazole, le sertaconazole, le kétoconazole, le posaconazole, le quilséconazole, l'otéséconazole (VT-1161) ou l'ibrexafungerp (SCY-078), en particulier le clotrimazole.

5. Emulsion selon l'une des revendications 1 à 4 **caractérisée en ce que** l'AINS est le diclofénac et celui-ci est contenu dans une gamme de concentrations de 0,2 - 0,4 % en poids de l'émulsion.

6. Emulsion selon l'une des revendications 1 à 5 dans laquelle est contenu un conservateur actif dans la gamme de pH de l'émulsion, de préférence où le conservateur est le phénoxyéthanol ou le propylèneglycol ou une combinaison de ces deux, ou le chlorure de déqualinium.

7. Emulsion selon l'une des revendications 1 à 6 **caractérisée en ce qu'**un antibiotique agissant contre les germes bactériens est contenu en outre, où l'antibiotique est de préférence la phosphomycine, la clindamycine, le métronidazole, la nitrofurantôine, la nitrofurazone, la nitrofurantôine, le nifuratel, la nifuroxacine, la nitroxoline, le triméthoprime, la sulfadiazine ou le cotrimoxazole.

8. Emulsion selon l'une des revendications 1 à 7 **caractérisée en ce qu'**un antiseptique est contenu en outre, de préférence en une quantité suffisante pour un effet antimicrobien aigu, où l'antiseptique est de préférence un sel d'ammonium quaternaire, de préférence choisi dans le groupe consistant en: le chlorure de benzalkonium, de préférence à une concentration d'au moins 0,2 % en poids; le chlorure de déqualinium, de préférence à une concentration d'au moins 0,2 % en poids; et le phénoxyéthanol, de préférence à une concentration d'au moins 2 % en poids.

9. Emulsion selon l'une des revendications 1 à 8 pour une utilisation dans le traitement des infections fongiques vaginales, en particulier des infections fongiques vaginales chroniques, de préférence où le traitement est un traitement topique.

10. Emulsion pour une utilisation selon la revendication 9 **caractérisée en ce que** l'infection fongique vaginale est une infection vaginale mixte par Candida albicans et des bactéries, comme Enterobacter, E. coli, Klebsiella pneumoniae, Gardnerella vaginalis, Prevotella spp..

11. Emulsion pour une utilisation selon la revendication 9 ou 10 **caractérisée en ce que** l'infection fongique vaginale est une mycose à candida, en particulier une candidose vulvo-vaginale.

12. Procédé de préparation d'une émulsion selon l'une des revendications 1 à 8 **caractérisé en ce que** l'AINS est introduit par le biais de la phase aqueuse lors de la préparation de l'émulsion.

13. Procédé de préparation d'une émulsion selon l'une des revendications 1 à 8, **caractérisé en ce que** l'AINS est introduit dans l'émulsion contenant l'antimycosique sous la forme d'un sel finement cristallin ou micronisé.

14. Procédé de préparation d'une émulsion selon l'une des revendications 1 à 8 **caractérisé en ce que** l'AINS est introduit dans l'émulsion contenant l'agent antimycosique par le biais d'un hydrogel.

15. Procédé de préparation d'une émulsion selon la revendication 7 ou 8 dans lequel l'addition des substances a lieu de telle manière qu'un effet antibactérien thérapeutiquement efficace est obtenu.
